# EUROPEAN PATENT SPECIFICATION

(11) **EP 2 568 286 B1**
(45) Date of publication and mention of the grant of the patent: **11.03.2015**
(21) Application number: 11777474.5
(22) Date of filing: 06.05.2011
(51) Int. Cl.: G01N 33/53, G01N 33/574

(54) **METHOD FOR ANALYSING MUCIN 1 HAVING SIA-ALPHA-2-8-SIA-ALPHA-2-3-GAL-BETA GLYCANS**
VERFAHREN ZUR ANALYSE VON MUCIN-1 ENTHALTEND SIA-ALPHA-2-8-SIA-ALPHA-2-3-GAL-BETA ZUCKERKETTEN
PROCÉDÉ D'ANALYSE DE MUCINE-1 AYANT UNE CHAÎNE SUCRE SIA-ALPHA-2-8-SIA-ALPHA-2-3-GAL-BETA

(30) Priority: 01.06.2010 JP 2010125766; 07.05.2010 JP 2010107294
(43) Date of publication of application: 13.03.2013
(73) Proprietor: Tokyo Institute of Technology, Tokyo 152-8550 (JP); Yamaguchi University, Yamaguchi 753-8511 (JP)
(72) Inventor: YAMASHITA, Katsuko, Yokohama-shi Kanagawa 226-8503 (JP); FUKUSHIMA, Keiko, Yokohama-shi Kanagawa 226-8503 (JP); HINODA, Yuji, Ube-shi Yamaguchi 755-8505 (JP)
(74) Representative: Gille Hrabal
(86) International application number: PCT/JP2011/060574
(87) International publication number: WO 2011/138955

(56) References cited:
- WO-A1-03/064655
- T. YUE ET AL: "The Prevalence and Nature of Glycan Alterations on Specific Proteins in Pancreatic Cancer Patients Revealed Using Antibody-Lectin Sandwich Arrays", MOLECULAR & CELLULAR PROTEOMICS, vol. 8, no. 7, 1 July 2009 (2009-07-01), pages 1697-1707, XP55076258, ISSN: 1535-9476, DOI: 10.1074/mcp.M900135-MCP200
- STORR S.J. ET AL.: 'The O-linked glycosylation of secretory/shed MUC1 from an advanced breast cancer patient's serum' GLYCOBIOLOGY vol. 18, no. 6, 2008, pages 456 - 462, XP055069218
- CAZET AURELIE ET AL.: 'GD3 synthase overexpression enhances proliferation and migration of MDA-MB-231 breast cancer cells' BIOL CHEM vol. 390, no. 7, 2009, pages 601 - 609, XP008164362
- RUCKHABERLE EUGEN ET AL.: 'Gene expression of ceramide kinase, galactosyl ceramide synthase and ganglioside GD3 synthase is associated with prognosis in breast cancer' J CANCER RES CLIN ONCOL vol. 135, no. 8, 2009, pages 1005 - 1013, XP019716003
- YAMASHIRO S ET AL.: 'Genetic and enzymatic basis for the differential expression of GM2 and GD2 gangliosides in human cancer cell lines' CANCER RESEARCH vol. 53, no. 22, 15 November 1993, pages 5395 - 5400, XP055069224
- KOH Y. ET AL.: 'Decreased expression of alpha2,8 sialyltransferase and increased expression of betal,4 N-acetylgalactosaminyltransferase in gastrointestinal cancers' EXPERIMENTAL BIOLOGY AND MEDICINE (MAYWOOD) vol. 227, no. 3, 2002, pages 196 - 200, XP055069233
- BYERS H L ET AL.: 'Isolation and characterisation of sialidase from a strain of Streptococcus oralis' J MED MICROBIOL vol. 49, no. 3, 2000, pages 235 - 244, XP055069237
- BRINKMAN-VAN DER LINDEN E C M ET AL.: 'New Aspects of Siglec Binding Specificities, Including the Significance of Fucosylation and of the Sialyl-Tn Epitope' J BIOL CHEM vol. 275, no. 12, 2000, pages 8625 - 8632, XP000907572
- MAYUMI HOSHINO ET AL.: 'Rinsho Tosa Bio Maker no Kaihatsu - Tosa Kino no Kaimei to sono Oyo, chapter 1, Gijutsu-hen, Tosa Kozo Kaiseki Gijutsu no Kaihatsu to Iryo Oyo ''3) Mucin no Glycosylation o Shihyo to shita Shippei Marker no Kanosei''' GENE & MEDICINE MOOK 2008, pages 40 - 45
- HIROKI TAKAHASHI ET AL.: 'Kanshitsusei Haien no Kessei Marker KL-6 no Saishin Chiken -SP-A, SP-D tono Kairi o Chushin ni - Dai 6 Kai Byotai kara Toraeta Kessei Marker Kairi no Kaishaku' RESPIRATORY MOLECULAR MEDICINE vol. 10, no. 2, 2006, pages 111 - 116, XP008168907
- PICCO GIANFRANCO ET AL.: 'Over-expression of ST3Gal-I promotes mammary tumorigenesis' GLYCOBIOLOGY vol. 20, no. 10, October 2010, pages 1241 - 1250, XP055069241
- NAOKI OHYABU ET AL: 'An Essential Epitope of Anti-MUC1 Monoclonal Antibody KL-6 Revealed by Focused Glycopeptide Library' JOURNAL OF THE AMERICAN CHEMICAL SOCIETY vol. 131, no. 47, 02 December 2009, pages 17102 - 17109, XP055001006 DOI: 10.1021/ja903361f ISSN: 0002-7863

### SEQUENCE LISTING

<110> TOKYO INSTITUTE OF TECHNOLOGY YAMAGUCHI UNIVERSITY
<120> Method of analyzing mucin 1 having sugar chain
<130> TIT-875
<150> JP 2010-107294
   <151> 2010-05-07
<150> JP 2010-125766
   <151> 2010-06-01
<160> 16 .
<170> PatentIn version 3.1
<210> 2
   <211> 3166
   <212> DNA
   <213> Homo sapiens
<400>
   cggagcggcg agtcggtgcc gcccgggctg cgcttcgccc cggcagcttt ggcggcgagg 60
   acgcccgtgg ctcaggatga gatcgggggg cacgctgttc gccctcatag gcagcctgat 120
   gctgctgctc ctcctgcgta tgctctggtg cccagccgac gcgcctgccc gctccaggct 180
   gttgatggag ggaagcagag aggacaccag tggtacctca gctgcactga agacactctg 240
   gagcccgaca accccggtac cacgcaccag gaacagcaca tatctggatg agaagacaac 300
   ccaaataaca gagaaatgca aagatctgca atatagcttg aactctttat ctaacaaaac 360
   gagacggtac tctgaggatg actacctcca gaccatcaca aacatacaga gatgcccatg 420
   gaaccggcaa gcagaagaat atgacaattt tagagcaaaa ctggcttcct gttgcgatgc 480
   cattcaagac ttcgtggttt cccagaacaa cactccagtg gggactaaca tgagctacga 540
   ggtggaaagc aagaaacaca tccccattcg agagaacatt ttccacatgt ttccagtgtc 600
   gcagcctttt gtggactatc cctataacca gtgtgcagtg gttggtaatg ggggaattct 660
   caacaagtct ctctgcggag cagaaattga taaatctgac ttcgtcttca ggtgtaacct 720
   ccccccaatc acagggagcg ctagtaaaga tgttggaagc aaaacaaatc ttgtgactgt 780
   caatcccagc attataaccc tgaagtacca gaatttgaag gagaagaaag cacagttttt 840
   ggaggacatc tccacctatg gagatgcatt cctcctcctg ccagcatttt cctatcgggc 900
   caacacaggc atctctttta aagtctacca aacactcaaa gagtcaaaaa tgaggcaaaa 960
   ggttctcttc ttccatccca ggtacctgag acacctcgct cttttctgga gaactaaagg 1020
   ggtgactgca taccgcttgt ccacaggctt gatgattgca agtgtcgctg tggaactgtg 1080
   tgaaaacgtg aagctctacg gattctggcc tttctctaag actatcgaag acaccccact 1140
   cagtcaccac tactatgata acatgttacc taagcatggt ttccaccaga tgcctaaaga 1200
   atacagccaa atgctccagc tccatatgag aggaatcctc aaactgcaat tcagcaaatg 1260
   tgaaacggct taacgtttct tagaaggaga ataatttcag gaggtggagt ggatgtgtca 1320
   cagcatctcc aaaaagccaa tagaagaagg cacagagaaa gcatgaatta caaaggcgct 1380
   ctcccacttg tctagaccaa agccacccgc ccccactcac tttgcagcct ccacgagtca 1440
   ctcattctca ccttcaacgt tctttctctg agaatagaga ccaaaacatc agacttggat 1500
   aagtaaaatg agataatttt tcaaatcatc atagaatttg atttgagcca gggtctctca 1560
   gaatgcttcc ttgttcctat ccatgatagc cattcccacc tttatcagag tggtaatgaa 1620
   actgtgcaat tgtgccaaag accctttctg aagagaatgt ctgaatcatg cgccgagttt 1680
   ttacacacag ctcttccttt ataaataaat ccttcccatt ctccctccta gtagagtaca 1740
   gaaacaaaat acccttgatg attcaggaag aaaagtcttt tttacttagc aatgtgcctg 1800
   cttctgattc agttcgcttg tgacattaag ctgggttggg gttttggttg gatttggggc 1860
   gtttcttcac ttcttttgtc tatattttcc ttacctttat cagtttgtat tcgagcttcc 1920
   tgctttggga ttctgcaatt ctctctccca ctgacaggat caactcaatg acataaagta 1980
   gttcaaacat ccattgcttc tcacatgttt tatccataaa gttactcatc tgattttatt 2040
   taaaatagtg aacatctact tgatatcaga cccgaggacc atcctccatt ggagaatatg 2100
   aagatattgt cactggcaga aaagcaggtg tgtgccatta attgataaga taccacaagc 2160
   atcatcatgc cagttatgaa cacagtgctg aaaggatcat agacaggggt ggttaaatct 2220
   gatcccagta gaataaactt cagtgtacct atttcaggga agagttaatt tcacaattaa 2280
   aactagtaaa tgaaccaatt cttaggcaca ttaagtggat tctgagtaaa agaaagggaa 2340
   cagcaggaga aagctgttcg cttggttctg attacccaaa tgagcatgct ggaaggaggt 2400
   tgtgaggcta cgctaaaacc tctgcgtagg gagagagtac agtgcatgag tgtggcggct 2460
   tttgtccaca ctcgtgaagg gtgagtaatt cagagccaat cacatcacaa ggatggacac 2520
   acctaactca tcacttcagg gggagatgaa tgctttcatg agaaattaca ctcataagct 2580
   aagcatcagt tttgagtaaa atttgagtag atgttaaata tgaacatttt atacctctta 2640
   ctaatgtccc accgacacct tttaatgtaa gcacatttat ttattaagtt acttgacatt 2700
   aaatgcttat gtctgtatat tctgttcatc catcgatttt cccaaaaagt aagagcatag 2760
   gagatgaggc ctacatgcca agaaaactat aaattttact ctttaattct tacttgagcc 2820
   agcttgttgt ttatcaagtg cttttttgaa gagacagcac cctgtgaatt cttcattctg 2880
   atacagtgtc accttgtatt taacatttgt aatgttgttt caagtttaca tctctttcat 2940
   tcttttatag caaatcaaac gtattagctt cagaaattta tcagaagttc atatataaat 3000
   attttgcaaa gggtaaaagg cttttttgtt aaataaaata aaatttatta ttttcttctg 3060
   atgaatagag gctcttttat gctgctgcta atgaacctaa ttagctttaa attatctcct 3120
   agcaacattg gtcacgtttc aatcatgcta ttagcaaaaa aaaaaa 3166
<210> 3
   <211> 21
   <212> DNA
   <213> Homo sapiens
<400> 3
   ggcaagcaga agaatatgca a 21
<210> 4
   <211> 24
   <212> DNA
   <213> Homo sapiens
<400> 4
   aaacaacaaa gttttgaaca gcat 24
<210> 5
   <211> 24
   <212> DNA
   <213> Homo sapiens
<400> 5
   ttcaacttac tctctcttcc caca 24
<210> 6
   <211> 22
   <212> DNA
   <213> Homo sapiens
<400> 6
   tcttcttcag aatcccacca tt 22
<210> 7
   <211> 23
   <212> DNA
   <213> Homo sapiens
<400> 7
   ctcagagatc gaagaagaaa tcg 23
<210> 8
   <211> 21
   <212> DNA
   <213> Homo sapiens
<400> 8
   gctgttcaca gctgatctga t 21
<210> 9
   <211> 21
   <212> DNA
   <213> Homo sapiens
<400> 9
   caggtaccca caaaacagtg c 21
<210> 10
   <211> 20
   <212> DNA
   <213> Homo sapiens
<400> 10
   gagcttactg ggtgccttgt 20
<210> 11
   <211> 23
   <212> DNA
   <213> Homo sapiens
<400> 11
   aatgtggaaa ggagattgac agt 23
<210> 12
   <211> 23
   <212> DNA
   <213> Homo sapiens
<400> 12
   tctgatttag ttcccacatc tgc 23
<210> 13
   <211> 20
   <212> DNA
   <213> Homo sapiens
<400> 13
   gctgaggcac gaaatattgg 20
<210> 14
   <211> 20
   <212> DNA
   <213> Homo sapiens
<400> 14
   tgtcgaacag ctctgactgc 20
<210> 15
   <211> 19
   <212> DNA
   <213> Homo sapiens
<400> 15
   atccacatcg ctcagacac 19
<210> 16
   <211> 19
   <212> DNA
   <213> Homo sapiens
<400> 16
   gcccaatacg accaaatcc 19

## Description

### TECHNICAL FIELD

The present invention relates to a method for distinguishing breast cancer from interstitial pneumonia characterized by analyzing mucin 1 having Siaα2-8Siaα2-3Galß-R.

### BACKGROUND ART

A "CA15-3" test, which is a clinical laboratory test, is used as a clinical marker of breast cancer. It is useful as an index of malignancy or degree of progress of breast cancer, and for monitoring the effects of treatment of breast cancer. The CA15-3 antigen is detected by an immunoassay method (sandwich assay) using a monoclonal antibody "115D8" against MAM-6, which is a glycoprotein on the human milk fat globule membrane and a monoclonal antibody "DF3" against liver tissue to which breast cancer spreads by metastasis. The CA15-3 antigen detected by the immunoassay method of the "CA15-3" test is an extracellular domain of mucin 1 of an epithelial cell.

On the other hand, it is known that a "KL-6" test which is a clinical laboratory tests, is useful as a diagnostic marker of interstitial pneumonia. The KL-6 antigen is detected by a sandwich assay using monoclonal antibody "KL-6". The monoclonal antibody "KL-6" is prepared by immunizing a VMRC-LCR cell line derived from lung adenocarcinoma (Non-patent literature 1). The monoclonal antibody "KL-6" recognizes a combination of amino acids sequences: PDTRPAP in mucin 1 and Neu5Acα2, 3Galβ1, 3GalNAcα residue which is a sialylated sugar chain bound to threonine

(Non-patent literature 2). Therefore, the KL-6 antigen detected by the sandwich assay of the "KL-6" test is also present in free mucin 1 which is a type of mucin 1.

As mentioned above, mucin 1 can be detected by both the immunoassay method of the "CA15-3" test for diagnostic markers of breast cancer and the immunoassay method of the "KL-6" test for diagnostic markers of interstitial pneumonia. Non-patent literature 3 discloses that the "KL-6" test is useful, as a diagnostic marker of breast cancer, for diagnostics of metastasis or relapse, and a judgment of the effect of treatment. That is to say, the "CA15-3" test and "KL-6" test are useful as diagnostic markers of both breast cancer and interstitial pneumonia.

However, breast cancer patients often suffer from interstitial pneumonia during therapy of breast cancer. As explained above, the "CA15-3" test is used as the monitoring of effect in treatment of breast cancer. If the breast cancer patient suffers from interstitial pneumonia during therapy of breast cancer, the measurement value of the CA15-3 antigen is increased. In this case, there is the problem that a metastasis or relapse of breast cancer cannot be distinguished from a development of interstitial pneumonia.

### CITATION LIST

### NON-PATENT LITERATURE

[Non-patent literature1] Laboratory Clinical Practice (Japan) 2003, vol.21, p.75-79
[Non-patent literature2] Journal of American Chemical Society (U.S.A) 2009, Vol.131, p.17102-17109
[Non-patent literature3] Nihon Rinsho Gekagakai Zashi (Japan) 2008, vol.69, p.1293-1302
[Non-patent literature4] Journal of Biological Chemistry (U.S.A) 2000, vol.275, p.15422-15431

### SUMMARY OF INVENTION

### TECHNICAL PROBLEM

The object of the present invention is to provide an analyzing method for distinguishing breast cancer from interstitial pneumonia.

### SOLUTION TO PROBLEM

The present inventor has conducted intensive studies seeking a tumor marker capable of distinguishing breast cancer from interstitial pneumonia, to surprisingly find that mucin 1 of breast cancer patients contains Siaα2-8Siaα2-3Galβ-R (α2,8-disialyl residue) which is a carbohydrate antigen. Further, the present inventor found an analyzing method which can specifically detect mucin 1 of breast cancer patients, using a probe capable of recognizing mucin 1 having α2,8-disialyl residue. Furthermore, according to the analyzing method of the present invention, all serum samples of breast cancer patients which have high levels of CA15-3, are positive, but all serum samples of interstitial pneumonia patients are negative. Therefore, it is possible to distinguish the detection of malignancy or degree of progress of breast cancer from the development of interstitial pneumonia by a serodiagnostic test.

The present invention is based on the above findings.

Namely, the present invention relates to:
[1] a method for distinguishing breast cancer from interstitial pneumonia by analyzing mucin 1 having Siaα2-8Siaα2-3Galβ-R, wherein the method comprises the step of bringing a first probe specifically binding to a mucin 1 having Siaα2-8Siaα2-3Galβ-R into contact with a sample to be tested,
[2] the method of the item [1], comprising the steps of: bringing the first probe specifically binding to the mucin 1 having Siaα2-8Siaα2-3Galβ-R into contact with the sample to be tested; and
   detecting a bound complex of the first probe and the mucin 1 having Siaα2-BSiaα2-3Galβ-R,
[3] the method of the item [1] or [2], wherein the method is a sandwich assay which comprises the steps of:
   bringing the first probe specifically binding to the mucin 1 having Siaα2-8Siaα2-3Galβ-R into contact with the sample to be tested;
   bringing a second probe specifically binding to the mucin 1 having Siaα2-8Siaα2-3Galβ-R into contact with the sample to be tested; and
   detecting the bound complex of the first probe and the mucin 1 having Siaα2-8Siaα2-3Galβ-R,
[4] the method for analyzing mucin 1 having Siaα2-8Siaα2-3Galβ-R of the items [1] to [3], wherein the first probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or a mixture thereof,
[5] the method of the item [3], wherein
   the first probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or a mixture thereof; and
   the second probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or an antibody specifically binding to the mucin 1, or an antibody fragment having the antigen-binding site thereof; or a mixture of two or more thereof.

The term "analyzing" or "analysis" as used herein includes a quantitative or semiquantitative measurement of an amount of a compound to be analyzed and a detection used to determine the presence or the absence of a compound to be analyzed.

### ADVANTAGEOUS EFFECTS OF INVENTION

Using the method for analyzing mucin 1 having Siaα2-8Siaα2-3Galβ-R of the present invention, it is possible to distinguish the detection of malignancy or degree of progress of breast cancer from the development of interstitial pneumonia.

### BRIEF DESCRIPTION OF THE DRAWINGS

FIG. 1 is a graph showing a standard curve of the mucin 1 having α2,8-disialyl residue by a sandwich assay.
FIG. 2 is a graph showing the expression of the mucin 1 having α2,8-disialyl residue in breast cancer cell lines (i.e. YMB-1 and MCF-7), gastric cancer cell line (i.e. NUGC-4), and lung cancer cell line (i.e. ABC-1).
FIG. 3 is a graph showing the measured results of mucin 1 having α2,8-disialyl residue, CA15-3, or KL-6 in sera of breast cancer patients, healthy persons, and interstitial pneumonia patients.
FIG. 4 is a view showing the main Siaα2-8Siaα2-3Galβ-R of MUC1 of breast cancer patients.
FIG. 5 is a photograph showing an increased expression of α2,8-sialyltransferase in a tissue of a breast cancer patient.

### DESCRIPTION OF EMBODIMENTS

### [1] Mucin 1 having Siaα2-8Siaα2-3Galβ-R [0011]

Mucin 1 is highly glycosylated type I transmembrane glycoprotein having a molecular weight of approximately 300 kD or more, which consists of a short N-terminal region, a center region, a transmembrane region, and a C-terminal cytoplasmic region. The center region contains a unique tandem repeat consisting of twenty amino acids (PDTRPAPGSTAPPAHGVTSA). Mucin 1 genes encoded in the center region are variable. That is, the number of tandem repeats in the region varies from 25 to 125. Further, mucin 1 genes have deletion, insertion, and substitution of amino acid(s), in addition to the variation of numbers of tandem repeats, and thus mucin 1 genes are highly variable. A part with variable numbers of tandem repeats in the center region is referred to as a VNTR region, and has many O-glycans. Whereas, oligopeptides in the juxtamembrane region other than the tandem repeats in the center region have N-glycans and O-glycans.

The mucin 1 having α2,8-disialyl residue analyzed in the method of present is not limited, as long as it has the α2,8-isialyl residue and the unique tandem repeat consisting of twenty amino acids (i.e. PDTRPAPGSTAPPAHGVTSA). The α2,8-disialyl residues are richly contained in the O-glycan of the VNTR region of the mucin 1 having α2,8-disialyl residue.

Amino acid sequences of mucin 1 contain an N-terminal region, a center region, a transmembrane region, and a C-terminal cytoplasmic region. However, the amino acid sequences of mucin 1 are variable, and thus, it is not limited to a specific amino acid sequence. Further, the number of the tandem repeats in mucin 1 is not limited, but is preferably 1 to 200, more preferably 5 to 150, more preferably 20 to 130, most preferably 25 to 125. Further, in the amino acids sequence of the N-terminal region, the center region (tandem repeats and the others), the transmembrane region, and the C-terminal cytoplasmic region, the amino acids sequence of the mucin 1 having α2,8-disialyl residue of the present invention may contain mutation(s). For example, one to 100 amino acids may be deleted, substituted, inserted, or added in the amino acid sequence of SEQ ID NO: 1.

In connection with this, if the mucin 1 having α2,8-disialyl residue analyzed in the method of the present invention is treated with sialidase, it cannot be recognized by the antibody specifically binding to α2,8-disialyl residue described below.

The molecular weight of the mucin 1 having α2, 8-disialyl residue analyzed in the method of the present invention is not limited, but it may be 20 to 10000kD. The mucin 1 having α2,8-disialyl residue, may be a membrane-bound one which is bound to membrane, a secretory one, or a partial peptide thereof. Therefore, body fluids of breast cancer patients can contain the membrane-bound mucin 1 having α2,8-disialyl residue, the secretory mucin 1 having α2,8-disialyl residue, or the partial peptide thereof.

Cells or tissue in which the mucin 1 having α2,8-disialyl residue analyzed in the method of the present invention is expressed, are also not limited, but it may be breast cancer cells, tissues of breast cancer, or cell lines derived from breast cancer. Until now, it has not been reported that mucin 1 expressed in cells of a living body has Siaα2-8Siaα2-3Galβ-R. For example, as shown in Examples, mucin 1 which is increased in bloods of interstitial pneumonia patients does not contain Siaα2-8Siaα2-3Galβ-R. Further, mucin 1 expresses in a lot of cancer cells. However, mucin 1 expressed in a gastric cancer cell line i.e. NUGC-4, or lung cancer cell line i.e. ABC-1, does not contain Siaα2-8Siaα2-3Galβ-R.

The mucin 1 having α2,8-disialyl residue may, for example, be isolated from specimens. In particular, the mucin 1 having α2,8-disialyl residue can be isolated from specimens derived from the living body (for example, blood such as serum of plasma, or breast cancer tissue), or established cell lines derived from breast cancer or culture supernatants thereof. The mucin 1 can be purified from the specimens using ammonium sulfate precipitation, ion-exchange column chromatography, hydrophobic column chromatography, gel filtration column chromatography, affinity column chromatography, dialysis, lyophilization, or the like. In particular, the mucin 1 can be purified using an affinity column prepared using an antibody specifically binding to the α2,8-disialyl residue or an antibody specifically binding to mucin 1 having α2,8-disialyl residue_{.}

The mucin 1 having α2,8-disialyl residue can be used as a standard substance in a method for analyzing mucin 1 having Siaα2-8Siaα2-3Galβ-R mentioned later. In addition, a kit for analyzing mucin 1 having Siaα2-8Siaα2-3Galβ-R may contain the mucin 1 having α2,8-disialyl residue of the present invention, as a standard substance.

Specifically, the Siaα2-8Siaα2-3Galβ-R (α2,8-disialyl residue) as used herein means Neu5Acα2→8Neu5Acα2→3Gal. A sugar chain containing α2,8-disialyl residue expressed in mucin 1 is not limited, but includes (A) Neu5Acα2→8Neu5Acα2→3Galβ1→4GlcNAcβ1→3Galβ1→3GalNAc→Ser(Thr) (hereinafter referred to as an α2,8-disialyl residue (A)) or (B) Neu5Acα2→8Neu5Acα2→3Galβ1→3GalNAc→Ser(Thr) (hereinafter referred to as an α2,8-disialyl residue (B)).

The mucin 1 derived from interstitial pneumonia patients does not have α2,8-disialyl residue (A) and α2,8-disialyl residue (B), but the mucin 1 derived from breast cancer patients expressα2,8-disialyl residue (A) and/or α2,8-disialyl residue (B).

The amount of α2,8-disialyl residue (A) in the mucin 1 having α2,8-disialyl residue varies from breast cancer patient to breast cancer patient. Thus, the amount of α2,8-disialyl residue (A) is not limited, but may be 0.1 to 99 % by weight, preferably 1 to 50 % by weight, more preferably 3 to 30 % by weight, most preferably 5 to 15 % by weight. Further, the amount of α2,8-disialyl residue (B) in the mucin 1 having α2,8-disialyl residue varies from breast cancer patient to breast cancer patient. Thus, the amount of α2,8-disialyl residue (B) is not limited, but may be 0.1 to 99 % by weight, preferably 0.1 to 10 % by weight, more preferably 0.3 to 5 % by weight, most preferably 0.3 to 3 % by weight.

The mucin 1 having α2,8-disialyl residue includes a mucin 1 having only α2,8-disialyl residue (A), a mucin 1 having only α2,8-disialyl residue (B), and mucin 1 having both α2,8-disialyl residue (A) and α2,8-disialyl residue (B).

The mucin 1 having α2,8-disialyl residue has sugar chains other than the α2,8-disialyl residue (A) and α2,8-disialyl residue (B). As the other sugar chain, there may be mentioned, for example, Neu5Acα2, 3Galβ1, Neu5Acα2→3Galβ1→3(Neu5Acα2→6)GalNAcα→Ser(Thr), and Neu5Acα2→3Galβ1→3(Neu5Acα2→3Galβ1→4GlcNAcβ1→6)GalNAcα→Ser(Th r).

### [2] Method for analyzing mucin 1 having Siaα2-8siaα2-3Galβ-R

The method for analyzing mucin 1 having α2,8-disialyl residue used in the method of the present invention is characterized by comprising (a) the step of bringing a first probe specifically binding to a mucin 1 having Siaα2-8Siaα2-3Galp-R into contact with a sample to be tested (hereinafter referred to as contact step (a)).

Further, the method for analyzing mucin 1 having α2,8-disialyl residue used in the method of the present invention may comprise (c) the step of detecting a bound complex of the first probe and the mucin 1 having Siaα2-8Siaα2-3Galβ-R (hereinafter referred to as detection step(c)), in addition to contact step (a).

Furthermore, the method for analyzing mucin 1 having α2,8-disialyl residue used in the method of the present invention may comprise (b) the step of bringing a second probe specifically binding to the mucin 1 having Siaα2-8Siaα2-3Galβ-R into contact with the sample to be tested (hereinafter referred to as contact step (b)), in addition to contact step (a) and the detection step.

As the first probe used in contact step (a), a "α2,8 mucin probe" specifically binding to mucin 1 having α2,8-disialyl residue can be used.

The "α2,8 mucin probe" may be, for example, an "α2,8-disialyl probe" specifically binding to α2,8-disialyl residue (for example, a lectin specifically binding to α2,8-disialyl residue, an antibody specifically binding to α2,8-disialyl residue, or an antibody fragment having antigen-binding site thereof); or an antibody specifically binding to the mucin 1 having α2,8-disialyl residue, or an antibody fragment having the antigen-binding site thereof.

As the second probe used in contact step (b), a "α2,8 mucin probe" specifically binding to mucin 1 having α2,8-disialyl residue, or a "universal mucin probe" specifically binding to mucin 1 with and without α2,8-disialyl residue can be used.

The "universal mucin probe" may be, for example, a lectin specifically binding to a sugar chain of mucin 1 other than α2,8-disialyl residue; or an antibody specifically binding to mucin 1, or an antibody fragment having antigen-binding site thereof.

The "α2,8 mucin probe" and the "universal mucin probe" will be explained in detail hereinafter.

### <<α2,8 mucin probe>>

### (Lectin specifically binding to α2,8-disialyl residue)

The lectin specifically binding to α2,8-disialyl residue is not limited as long as it can bind to α2,8-disialyl residue. For example, the lectin specifically binding to α2,8-disialyl residue includes a lectin specifically binding to Siaα2-8Siaα2-3Galβ-R, or lectin specifically binding to Siaα2-8Sia.

### (Antibody specifically binding to α2,8-disialyl residue)

The antibody specifically binding to α2,8-disialyl residue is not limited as long as it can bind to α2,8-disialyl residue. Antibodies specifically binding to α2,8-disialyl residue include, for example, an antibody specifically binding to Siaα2-8Siaα2-3Galβ-R, or an antibody specifically binding to Siaα2-8Sia. The antibody specifically binding to α2,8-disialyl residue can bind to α2,8-disialyl residue alone, and therefore, can also bind to a glycoprotein or glycolipid having α2,8-disialyl residue.

The antibody specifically binding to α2,8-disialyl residue can be prepared by a known method except that the α2,8-disialyl residue or the glycoprotein having α2,8-disialyl residue is used as an immunizing antigen. For example, the monoclonal antibody can be prepared according to Koehler and Milstein's method (Nature 256: 495-497, 1975). In addition, the polyclonal antibody can be prepared by conventional immunization with an antigen that is α2,8-disialyl residue or glycoprotein having α2,8-disialyl residue alone or conjugated to BSA, KLH or the like, which is mixed with an adjuvant such as Freund's complete adjuvant, for example, in the skin of a rabbit. The blood is collected when the antibody titer increases, and may be used as it is as an antiserum, or the antibody may be used after purification by a known method.

Specifically, as the antibody specifically binding to α2,8-disialyl residue, a monoclonal antibody S2-566 and a monoclonal antibody 1E6 described in Non-patent literature 4 may be used. The monoclonal antibody S2-566 was obtained by immunization with human SK-MEL-28 melanoma cell line as an antigen, and specifically binds to Siaα2-8Siaα2-3Galβ-R (Neu5Acα2→8Neu5Acα2→3Gal). The monoclonal antibody 1E6 specifically binds to Siaα2-8Sia

### (Antibody specifically binding to mucin 1 having α2,8-disialyl residue)

The antibody specifically binding to mucin 1 having α2,8-disialyl residue binds the mucin 1 having α2,8-disialyl residue, but does not bind a mucin 1 without α2,8-disialyl residue. In addition, it does not bind to α2,8-disialyl residue without mucin 1. That is to say, the antibody recognizes a combination of α2,8-disialyl residue and a peptide (some amino acids) of mucin 1.

The antibody specifically binding to mucin 1 having α2,8-disialyl residue can be prepared by a known method, except that the mucin 1 having α2,8-disialyl residue is used as an immunizing antigen. For example, the monoclonal antibody can be prepared according to Koehler and Milstein's method (Nature 256: 495-497, 1975). The antibody specifically binding to mucin 1 having α2,8-disialyl residue can be obtained by screening a hybridoma producing a monoclonal antibody capable of specifically binding to mucin 1 having α2,8-disialyl residue, and incapable of binding to mucin 1 without α2,8-disialyl residue and the α2,8-disialyl residue without mucin 1. In addition, the polyclonal antibody can be prepared by conventional immunization with an antigen that is mucin 1 having α2,8-disialyl residue alone or conjugated to BSA, KLH or the like, which is mixed with an adjuvant such as Freund's complete adjuvant, for example, in the skin of a rabbit. The blood is collected when the antibody titer increases, and then an antibody binding to mucin 1 without α2,8-disialyl residue, and an antibody binding to α2,8-disialyl residue without mucin 1, are absorbed by an affinity column, or the like, and the polyclonal antibody specifically binding to mucin 1 having α2,8-disialyl residue can be obtained.

### <<Universal mucin probe>>

### (Lectin specifically binding to a sugar chain of mucin 1 other than α2,8-disialyl residue)

The lectin specifically binding to a sugar chain of mucin 1 other than α2,8-disialyl residue is not limited as long as it can bind to the sugar chain of mucin 1 other than α2,8-disialyl residue. For example, a lectin (such as Jacalin) specifically binding to a sialylated sugar chain i.e. Neu5Acα2,3Galβ1,3GalNAcα which is recognized by an after-mentioned monoclonal antibody KL-6 may be used.

### (Antibody specifically binding to mucin 1)

The antibody specifically binding to mucin 1 is not limited as long as it can bind to mucin 1 with and without α2,8-disialyl residue. Examples include, an antibody capable of binding to mucin 1 with and without α2,8-disialyl residue, and incapable of binding to α2,8-disialyl residue without mucin 1. In particular, the antibody specifically binding to mucin 1 includes conventional antibodies binding to mucin 1.

The antibody specifically binding to mucin 1 can be prepared by a known method except that the mucin 1 is used as an immunizing antigen. For example, the monoclonal antibody can be prepared according to Koehler and Milstein's method (Nature 256: 495-497, 1975). In addition, the polyclonal antibody can be prepared by conventional immunization with an antigen that is mucin 1 alone or conjugated to BSA, KLH or the like, which is mixed with an adjuvant such as Freund's complete adjuvant, for example, in the skin of a rabbit. The blood is collected when the antibody titer increases, and may be used as it is as an antiserum, or the antibody may be used after purification by a known method.

In particular, the antibody specifically binding to mucin 1 includes the monoclonal antibody KL-6 described in Non-patent literature 2. The monoclonal antibody KL-6 was obtained by immunization with human lung cancer cell line i.e. VMRC-LCR as an antigen, and specifically binds to mucin 1. The monoclonal antibody KL-6 recognizes a combination of an amino acids sequence i.e. PDTRPAP in mucin 1 and Neu5Acα2,3Galβ1,3GalNAcα residue which is sialylated sugar chain bound to threonine.

Further, in addition to the monoclonal antibody KL-6 many anti-mucin 1 monoclonal antibodies, are commercially available. Anti-mucin 1 polyclonal antibody or monoclonal antibody sold by, for example, Abcam or CTS, can be used as the antibody specifically binding to mucin 1.

### «Embodiments of the analyzing method of the present invention»

The method of the present invention is not limited, but includes the following embodiments.

The first embodiment of the method for analyzing mucin 1 having α2,8-disialyl residue used in the method of the invention comprises contact step (a), contact step (b), and optionally detection step (c). In the analyzing method of the present invention, either contact step (a) or contact step (b) may be carried out first. Thus, the first embodiment includes the following two examples differing in the order of contact step (a) and contact step (b):
(1) The first example is carried out in the order: contact step (a), contact step (b) and optionally detection step (c).
(2) The second example is carried out in the order: contact step (b), contact step (a) and optionally detection step (c).

For example, in the case of carrying out the analyzing method of the present invention by sandwich assay using antibodies as the probe, it can include:
(1) a sandwich assay wherein:
   (a) the step of bringing the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or a mixture of two or more thereof; into contact with the sample to be tested;
   (b) the step of bringing the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or the antibody specifically binding to mucin 1, or the antibody fragment the having antigen-binding site thereof; or the mixture of two or more thereof; into contact with the sample to be tested; and
   (c) the step of detecting a bound complex of the antibody and the mucin 1 having α2,8-disialyl residue; are carried out in the sequential order (hereinafter referred to as a sandwich assay (1)); and
(2) a sandwich assay wherein
   (b) the step of bringing an antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or the antibody specifically binding to the mucin 1, or the antibody fragment having the antigen-binding site thereof; or the mixture of two or more thereof; into contact with the sample to be tested;
   (a) the step of bringing the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or a mixture of two or more thereof; into contact with the sample to be tested; and
   (c) the step of detecting a bound complex of the antibody and the mucin 1 having α2,8-disialyl residue; are carried out in the sequential order (hereinafter referred to as a sandwich assay (2)).

For example, the sandwich immunoassay can be carried out according to the following procedure.

### (i) First reaction process

A capture antibody (first antibody) or an antibody fragment is immobilized to an appropriate insoluble carrier, such as a microtiter plate or a micro bead. Then the insoluble carrier is coated with an appropriate blocking agent, such as bovine serum albumin (BSA) or gelatin, to prevent a non-specific binding of the sample to the insoluble carrier. Thereafter, the sample which may contain mucin 1 having α2,8-disialyl residue, and first reaction buffer are added to the microtiter plate or the micro bead. Then mucin 1 having α2,8-disialyl residue in the sample is brought into contact with the capture antibody, to perform a reaction.

### (ii) Second reaction process

Then, a labeled antibody (second antibody) in which an antibody binding to the mucin 1 having α2,8-disialyl residue is conjugated to an enzyme such as horseradish peroxidase (HRP), is added to the whole, so as to bind the labeled antibody to the captured antigen (mucin 1 having α2,8-disialyl residue), and form an immune complex (i.e. the capture antibody/mucin 1 having α2,8-disialyl residue/labeled antibody complex) on the insoluble carrier such as the microtiter plate.

Further, a "biotin labeled antibody" or an "unlabeled" antibody" as the second antibody, can be used instead of the above "labeled antibody," which is labeled with an enzyme.

### (iii) Detection process

The insoluble carrier, such as the microtiter plate or the micro bead is washed with an appropriate wash buffer, and then a colorimetric substrate or a luminescent substrate for the enzyme of the labeled antibody is added. A detectable signal may be developed by a reaction of the enzyme and the substrate.

Alternatively, if the unlabeled antibody is used instead of the directly labeled second antibody, a labeled antibody, which may bind to the second antibody, can be used to detect the signal. Further, if the biotin labeled antibody is used, an enzyme labeled by avidin can be used so as to detect the signal.

In the case of the sandwich assay (1), the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or a mixture of two or more thereof is used as the capture antibody (first antibody); and the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or the antibody specifically binding to the mucin 1, or the antibody fragment having the antigen-binding site thereof; or the mixture of two or more thereof is used as the labeled antibody (second antibody).

Further, in the case of sandwich assay (2), the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or the antibody specifically binding to the mucin 1, or the antibody fragment having the antigen-binding site thereof; or a mixture of two or more thereof is used as the capture antibody (first antibody); and the antibody specifically binding to α2,8-disialyl residue, or the antibody fragment having the antigen-binding site thereof; the antibody specifically binding to the mucin 1 having α2,8-disialyl residue, or the antibody fragment having the antigen-binding site thereof; or a mixture of two or more thereof is used as the labeled antibody (second antibody).

The mucin 1 having α2,8-disialyl residue has tandem repeats, and thus, the mucin 1 having α2,8-disialyl residue may have multiple identical epitopes in a single molecule. Therefore, an antibody binding to the same epitope can be used both as the capture antibody (first antibody) and the labeled antibody (second antibody) in the sandwich assay system. In particular, the antibody specifically binding to α2,8-disialyl residue, i.e. the monoclonal antibody S2-566, can be used both as the capture antibody and the labeled antibody. In addition, the antibody specifically binding to Siaα2-8Sia, i.e. the monoclonal antibody 1E6, can be used both as the capture antibody and the labeled antibody.

The sandwich assay can be carried out by enzyme immunoassay, chemiluminescent immunoassay, or radioimmunoassay. Therefore, examples of the enzyme that labels the antibody include horseradish peroxidase (HRP), alkaline phosphatase, β-galactosidase, and luciferase. Furthermore, in addition to the enzyme, luminescent substances such as acridinium derivatives, fluorescent substances such as europium, radioactive substances such as I¹²⁵, and the like may be used as a label substance. In addition, the substrate and the luminescent inducer may be properly selected in accordance with the label substance. Furthermore, the labeled antibody in the present invention may also include an antibody which is bound to a substance such as hapten or low molecular weight peptide as a detection marker, or lectin that may be used in the signal detection of the antigen-antibody reaction.

A second embodiment of the method of the invention comprising analyzing mucin 1 having α2,8-disialyl residue comprises contact step (a), and detection step (c), but not contact step (b).

Specifically, (a) the step of bringing the "α2,8 mucin probe" specifically binding to the mucin 1 having α2,8-disialyl residue into contact with the sample to be tested, is carried out, and then (c) the step of detecting the bound complex of the "α2,8 mucin probe" and the mucin 1 having α2,8-disialyl residue, is carried out. As the "α2,8 mucin probe", the lectin specifically binding to α2,8-disialyl residue, the antibody specifically binding to α2,8-disialyl residue, the antibody specifically binding to mucin 1 having α2,8-disialyl residue, or the like, can be used.

In the case of using the antibody as the "α2,8 mucin probe", the second embodiment can be carried out by, for example, latex agglutination immunoassay, a fluorescent antibody method, radioimmunoassay, an immunoprecipitation method, an immunohistological staining method, the western blot, or the like. When lectin is used as the "α2,8 mucin probe", the second embodiment can be carried out by a lectin blotting technique.

A third embodiment of the method of the invention using analyzing mucin 1 having α2,8-disialyl residue comprises contact step (a), but not contact step (b) and detection step(c). In particular, the third embodiment includes a method wherein the mucin 1 having α2,8-disialyl residue is bound to the α2,8 mucin probe and then the mucin 1 having α2,8-disialyl residue is dissociated from the α2,8 mucin probe and collected. When the lectin specifically binding to α2,8-disialyl residue is used as the α2,8 mucin probe, a lectin affinity column can be used in the third embodiment. Further, in the case of using the antibody specifically binding to α2,8-disialyl residue or mucin 1 having α2,8-disialyl residue as the α2,8 mucin probe, an antibody affinity column can be used in the third embodiment.

The mucin 1 having α2,8-disialyl residue in the sample is bound to the lectin affinity column or the antibody affinity column, and eluted therefrom, whereby only the mucin 1 having α2,8-disialyl residue is collected. The collected mucin 1 having α2,8-disialyl residue can be detected by a general protein detecting method, such as protein staining after gel electrophoresis, or protein detection by UV meter; or a detecting method specific for mucin 1, such as an enzyme immunoassay of the "KL-6" test or the "CA15-3" test.

### (Samples to be tested)

Examples of the sample used in the analyzing method of the present invention include biological samples derived from the human body possibly containing mucin 1 having α2,8-disialyl residue. Examples of the sample to be tested include: urine, blood, serum, plasma, spinal fluid, saliva, cells, tissue or organ, and preparations thereof (for example, a biopsy sample, particularly a biopsy sample of breast cancer patient). The sample to be tested is preferably blood, serum, plasma, or a biopsy sample of a lacteal gland, particularly preferably blood, serum, or plasma. Blood, serum, or plasma is appropriate as a sample to be tested for detecting the breast cancer, because mucin 1 having α2,8-disialyl residue is released into the blood in breast cancer patients, whereas little mucin 1 having α2,8-disialyl residue exists in the blood, serum, or plasma of normal healthy subjects.

A liquid sample such as urine, blood, serum, plasma, spinal fluid and saliva may be used diluted with an appropriate buffer depending on the analysis method. In addition, a solid sample such as cells, tissue or organ is homogenized with an appropriate buffer in the amount of about 2 to 10 times the volume of the solid sample, and a suspension or a supernatant thereof may be used in the analysis method as it is, or after further dilution.

The "biological samples derived from the human body possibly containing mucin 1 having α2,8-disialyl residue" as used herein include samples containing mucin 1 and samples possibly containing mucin 1. This is because a sample derived from a patient possibly suffering from breast cancer is sometimes used in the analyzing method.

### [3] Method for distinguishing breast cancer from interstitial pneumonia

According to the method of the invention using analyzing mucin 1 having α2,8-disialyl residue, it is possible to distinguish breast cancer from interstitial pneumonia.

It is possible to distinguish whether the subject (patient) suffers from breast cancer or interstitial pneumonia, by measuring mucin 1 having α2,8-disialyl residue in the subject (patient) using the method for analyzing mucin 1 having α2,8-disialyl residue, and comparing the amounts measured to the amounts of mucin 1 having α2,8-disialyl residue found in interstitial pneumonia patients.

In the method of the present invention, a first probe specifically binding to the mucin 1 having α2,8-disialyl residue can be employed.

As the first probe, the "α2,8 mucin probe" specifically binding to mucin 1 having α2,8-disialyl residue can be used. The "α2,8 mucin probe": the "α2,8-disialyl probe" specifically binding to α2,8-disialyl residue (for example, the lectin specifically binding to α2,8-disialyl residue, the antibody specifically binding to α2,8-disialyl residue, or the antibody fragment having the antigen-binding site thereof); or an antibody specifically binding to the mucin 1 having α2,8-disialyl residue, or an antibody fragment having the antigen-binding site thereof, or a mixture of two or more thereof.

In the method of the invention, further a second probe specifically binding to mucin 1 having α2,8-disialyl residue. The "α2,8 mucin probe" specifically binding to mucin 1 having α2,8-disialyl residue can be used, or the "universal mucin probe" specifically binding to mucin 1 with and without α2,8-disialyl residue, can be used as the second probe. The "universal mucin probe", may be: the lectin specifically binding to a sugar chain of mucin 1 other than α2,8-disialyl residue; or the antibody specifically binding to mucin 1, or the antibody fragment having the antigen-binding site thereof

The first probe and second probe may be bound to a carrier, or may be dissolved in buffer solution. Examples of the carrier include: sepharose, cellulose, agarose, dextran, polyacrylate, polystyrene, polyacrylamide, polymethacrylamide, copolymer of styrene and divinylbenzene, polyamide, polyester, polycarbonate, polyethyleneoxide, hydroxypropyl methylcellulose, polyvinyl chloride, polymethylacrylate, copolymer of polystyrene and polystyrene, polyvinyl alcohol, polyacrylic acid, collagen, calcium alginate, latex, polysulfone, silica, zirconia, alumina, titania and ceramics. The form of the carrier is not also particularly limited, but includes particulate beads, microtiter plates, gel and the like.

For example, in the case where an immunological technique using a labeled antibody is used, such as an enzyme immunoassay, chemiluminescence, fluorescent antibody method, or radioactivity, the first antibody can be used in the form of a labeled antibody or a labeled antibody fragment conjugated with a labeling substance. The second antibody may be used in the form of a labeled antibody or a labeled antibody fragment conjugated with a labeling substance. Specific examples of the labeling substance include enzymes such as peroxidase (HRP), alkaline phosphatase (ALP), β-D-galactosidase or glucose oxidase, fluorescent substances such as fluorescein isothiocyanate or rare-earth metal chelates, radioactive isotopes such as ³H, ¹⁴C or ¹²⁵I, and, miscellaneously, biotin, avidin, and chemiluminescent substances. In the case where antibodies labeled with enzymes such as HRP, ALP or the like are used, they preferably contain an appropriately selected substrate and
the like since they cannot generate a measurable signal by themselves.

### [4] Method for detecting breast cancer by analyzing glycosyltransferase (not according to the invention)

As shown in Example 5, an increase of expression of α2,8-sialyltransferase I to VI, particularly α2,8-sialyltransferase III or VI (hereinafter sometimes referred to as ST8-III or ST8-VI, respectively) in breast cancer, strongly relates to the increase of the α2,8-disialyl residue in mucin 1 of breast cancer. Thus, an analysis of α2,8-sialyltransferase in a sample derived from a breast cancer patient makes it possible to distinguish breast cancer patients from normal healthy subjects. The method for detecting breast cancer using ST8-VI which is one of the α2,8-sialyltransferases, now will be further illustrated below. However the α2,8-sialyltransferase is by no means limited to ST8-VI.

In the detection method, the method of analyzing ST8-VI is not particularly limited as long as the method allows detection of ST8-VI quantitatively or semi-quantitatively, or the method allows determination of the presence or absence of ST8-VI. Examples of the method of analyzing ST8-VI include molecular biological assays of measuring the mRNA amount of ST8-VI (for example, the southern blot method, the northern blot method, and PCR method), immunological techniques using an antibody for ST8-VI or a fragment thereof (for example, enzyme immunoassay, latex agglutination immunoassay, chemiluminescent immunoassay, a fluorescent antibody method, radioimmunoassay, an immunoprecipitation method, an immunohistological staining method, or the western blot), and biochemical techniques (for example, enzymological assay).

### <<Molecular biological assay>>

The molecular biological assay for ST8-VI is not particularly limited as long as it is an assay using primers and probes that can hybridize to genes such as mRNA or cDNA obtained from ST8-VI and nucleotides thereof, in a sample on the basis of the principle of hybridization. For example, the molecular biological assay for ST8-VI includes the southern blot method, the northern blot method, or a PCR method. However, a reverse transcription-PCR (RT-PCR) is preferably used and a real-time RT-PCR is more preferably used.

Examples of the real-time PCR method include: the intercalator method in which a primer set composed of a forward primer and a reverse primer is used, and an intercalator such as SYBR Green I, which is a compound producing fluorescence by binding to a double strand DNA, is added to the PCR reaction system; and the TaqMan method in which the primer set, and a probe of which the 5' terminal is modified with a reporter pigment and the 3' terminal is modified with a quencher pigment (TaqMan probe), are added to the PCR reaction system. Such real-time PCR methods are well known, and kits and apparatus therefor are commercially available, and thus the real time PCR method can be easily conducted using commercially available kits and apparatus if the primer set, or the primer set and the probe are synthesized.

The forward primer, the reverse primer and the probe can be synthesized on the basis of the base sequences of the nucleotides that encode ST8-VI. Specifically, the forward primer and reverse primer, and the probe for ST8-VI can be synthesized by selecting appropriate base sequences from the base sequences (GenBank accession no. 338596) of cDNA that encodes ST8-VI represented by SEQ ID NO: 2. For example, the base sequence for the forward primer is 5'-GGCAAGCAGAAGAATATGCAA -3' (SEQ ID NO: 3) and the base sequence for the reverse primer is 5'-AAACAACAAAGTTTTGAACAGCAT -3' (SEQ ID NO: 4).

The length of the primer is not particularly limited, but is preferably 15-mer to 35-mer, more preferably 16-mer to 30-mer, and most preferably 19-mer to 25-mer. The length of the probe is not necessarily limited, but preferably 12-mer to 30-mer, more preferably 13-mer to 29-mer, and most preferably 14-mer to 18-mer.

The PCR method, particularly the real-time PCR method may include:
(1) a process of purifying mRNA from a sample derived from the human body,
(2) a process of synthesizing cDNA using a reverse transcription enzyme with the purified mRNA as a template,
(3) a process of amplifying DNA using a primer set, or a primer set and a probe, and
(4) a process of detecting the amplified DNA.

It is possible to determine whether a patient suspected of suffering from breast cancer has breast cancer or not by measuring the expression level of mRNA of ST8-VI in a sample derived from the body of the patient, and comparing the resulting expression level with the expression level of ST8-VI in a sample derived from the body of a normal healthy subject. More specifically, if the expression level of ST8-VI of the suspected patient is significantly more than the expression level of ST8-VI of the normal healthy subject, it is possible to determine that the patient has breast cancer.

For example, in the case of the real-time PCR in Examples described below, the average value of ST8-VI in normal healthy subjects is calculated, and then the standard deviation (SD) is calculated. The cutoff value for detecting breast cancer in patients is not limited as long as it is a value that allows detection of breast cancer. For example, a sample having a value higher than the average value may be determined as positive, or the average value +SD, average value +2SD, or average value +3SD may be taken as the cutoff value.

### «Immunological assay»

In the case where an immunological assay is used as the method for the analysis of ST8-VI, a monoclonal antibody or a polyclonal antibody binding to ST8-VI may be used.

The monoclonal antibody or the polyclonal antibody can be prepared by a known method except that ST8-VI is used as an immunizing antigen. For example, the monoclonal antibody can be prepared according to Koehler and Milstein's method (Nature 256: 495-497, 1975). In addition, the polyclonal antibody can be prepared by conventional immunization with an antigen that is ST8-VI alone, or conjugated to BSA, KLH or the like, which is mixed with an adjuvant such as simple adjuvant or Freund's complete adjuvant, for example, in the skin of a rabbit. The blood is collected when the antibody titer increases, and may be used as it is as an antiserum, or the antibody may be used after purification by a known method.

In the case where an enzyme immunoassay, particularly the sandwich assay is used as the immunological assay, it may be performed as described below.

Antibody binding to ST8-VI (capture antibody or first antibody) is immobilized onto an insoluble carrier such as a microtiter plate and bead. Then, blocking of the insoluble carrier is performed with an appropriate blocking agent (for example, bovine serum albumin or gelatin) in order to prevent non-specific binding onto the capture antibody or the insoluble carrier. To the microtiter plate and bead onto which the capture antibody is immobilized, a sample to be tested containing ST8-VI is added together with a first reaction solution, to bring the capture antibody into contact with ST8-VI for binding (first reaction process). Then, antigens and foreign substances that are not bound to the capture antibody are washed away with an appropriate washing solution (for example, a phosphate buffer containing a surfactant). Next, a labeled antibody (second antibody), in which an antibody binding to the captured ST8-VI is bound to an enzyme such as horseradish peroxidase (HRP), is added, to bind the labeled antibody to the captured antigen (second reaction process). By this reaction, an immune complex of the capture antibody, ST8-VI and the labeled antibody is formed on the carrier. The unbound labeled antibody is washed away with a washing solution, and a chromogenic substrate and a luminescent substrate for the enzyme of the labeled antibody are added to the immune complex, and then the signal is detected. In addition, it is also possible to detect a signal by labeling an antibody binding to the second antibody without directly labeling the second antibody.

Examples of the enzyme that labels the antibody include horseradish peroxidase (HRP), alkaline phosphatase, β-galactosidase, and luciferase. Furthermore, in addition to the enzyme, luminescent substances such as acridinium derivatives, fluorescent substances such as europium, radioactive substances such as I¹²⁵ , and the like may be used as a label substance. In addition, the substrate and the luminescent inducer may be properly selected in accordance with the label substance. Furthermore, the labeled antibody used in the method of the present invention may also include an antibody which is bound to a substance such as hapten or low molecular weight peptide as a detection marker, or lectin that may be used in the signal detection of the antigen-antibody reaction.

Furthermore, in the case where a biopsy sample of lacteal gland is used as a sample to be tested, it is possible to determine whether the sample is diagnosed as breast cancer or not by checking expression of ST8-VI at the lacteal gland by an immunohistological staining method using monoclonal antibody or polyclonal antibody.

In addition, in the case where blood or the like is used as a sample to be tested, it is possible to determine whether a patient suspected suffering from breast cancer has breast cancer or not by collecting the blood from the patient and using the whole blood itself, or as serum or plasma, measuring the amount of ST8-VI in the blood, and comparing that with the amount of ST8-VI in the blood or the like collected from normal healthy subjects. More specifically, if the amount of ST8-VI of the patient is significantly higher than the amount of ST8-VI of normal healthy subjects, it can be determined that the patient has breast cancer.

For example, in the case of the sandwich ELISA assay, the average value of ST8-VI in normal healthy subjects was calculated, and then the standard deviation (SD) was calculated. The cutoff value for detecting breast cancer in patients is not limited as long as it is a value that allows detection of breast cancer. For example, a sample having a value higher than the average value may be determined as positive, or the average value ±SD, average value ±2SD, or average value ±3SD may be taken as the cutoff value.

Examples of the sample used in the analysis of ST8-VI in the method for detecting breast cancer by the analysis of glycosyltransferase of the present invention, include biological samples and samples derived from the human body possibly containing ST8-VI. Specific examples of the sample to be tested include body fluid samples such as urine, blood, serum, plasma, spinal fluid and saliva, cells, tissue, organ, and preparations thereof (for example, a biopsy sample, particularly a biopsy sample of lacteal grand). The sample to be tested is preferably blood, serum, plasma, or a biopsy sample of lacteal grand, particularly preferably blood, serum, or plasma (hereinafter, sometimes referred to as "blood or the like"). Blood, serum or plasma are appropriate as samples to be tested for detecting breast cancer, because little ST8-VI exists in the tissue, blood, serum or plasma of normal healthy subjects.

### [5] Detection kit for breast cancer by the analysis of glycosyltransferase (not according to the invention)

The kit for detecting breast cancer by molecular biological analysis may contain a primer set, or a primer set and a probe that hybridize specifically to nucleotides that encode α2,8-sialyltransferase, particularly ST8-VI. In the detection kit, a forward primer, a reverse primer, and a probe may be contained as a mixture, or may be contained as separate reagents. In addition, the kit may further contain reagents and/or enzymes that are necessary in performing the real-time PCR method, in addition to the primers and the probe. Furthermore, the kit may contain a manual that describes use for detection or measurement of breast cancer, or use for differentiation of patients with breast cancer from normal healthy subjects. In addition, these descriptions may be also attached to the container of the kit.

The kit for detecting breast cancer by immunological analysis may contain an antibody that specifically binds to α2,8-sialyltransferase, particularly ST8-VI or a fragment thereof in a desired form depending on the immunological technique to be used. As the antibody, a monoclonal antibody or a polyclonal antibody may be used. The antibody fragment is not particularly limited as long as it has the ability to specifically bind to ST8-VI, examples include, for example, Fab, Fab', F(ab')₂, or Fv.

For example, in the case where an immunological technique using a labeled antibody is used, such as an enzyme immunoassay, chemiluminescence, a fluorescent antibody method, or radioactivity, the diagnosis kit may contain the antibody or a fragment thereof in the form of a labeled antibody or a labeled antibody fragment conjugated with a label substance. Concrete examples of the label substance include enzymes such as peroxidase, alkaline phosphatase, β-D-galactosidase or glucose oxidase, fluorescent substances such as fluorescein isothiocyanate or rare-earth metal chelates, radioactive isotopes such as ³H, ¹⁴ C or ¹²⁵I, and, miscellaneously, biotin, avidin, and chemiluminescent substances. In the case where the enzyme or the chemiluminescent substance is used, the kit preferably contains an appropriately selected substrate and the like since the enzyme or the chemiluminescent substance cannot generate measurable signals by themselves.

Furthermore, the kit may contain a manual that describes use for detection or measurement of breast cancer, or use for differentiation of patients with breast cancer from normal healthy subjects. In addition, these descriptions may be also attached to the container of the kit.

### EXAMPLES

The present invention will now be further illustrated by, but is by no means limited to, the following Examples.

### «Reference Example 1: Isolation of mucin 1 having α2,8. disialyl residue»

In this Reference Example, mucin 1 having α2,8-disialyl residue was isolated and purified from a breast cancer cell line.

The breast cancer cell line YMB-1 (1×10⁷ cells) was collected by centrifugation. 1 mL of an extraction buffer (50mM Tris-HCl, 0.15M NaCl, pH8.0, 1.0%NP-40) was added to the collected cells and the cells were lysed by vigorous stirring. The resulting solution was centrifuged at 15,000g for 15 minutes to collect a supernatant. 1 mL of the supernatant was applied to an affinity column with KL-6 antibody, which was equilibrated with the extraction buffer, and was stood for 30 minutes. The affinity column was washed with a 20-fold volume of the extraction buffer, and further washed with a 5-fold volume of PBS. Mucin 1 was eluted with 3 mL of an elution buffer (10mM KH₂PO₄, 3M NaCl, pH2.5) to obtain a mucin 1 solution.

The above affinity column with KL-6 antibody was prepared by using CNBr-Sepharose (GEhealthcare), in accordance with the protocol recommended by the manufacturer.

### <<Reference Example 2: Construction of measurement system of mucin 1 having α2,8-disialyl residue>>

In this Reference Example, an immunological assay system for mucin 1 having α2,8-disialyl residue was constructed by a sandwich assay.

50 µL of a monoclonal antibody S2-566 diluted to a concentration of 1µg/mL with PBS, was added to a 96-well black high-binding plate (Corning Inc., Corning, NY), and was immobilized at 4 °C for 16 hr. The surface of each well was blocked with PBS containing 1 % BSA at 25 °C for 1 hr. As a standard substance, the solution of YMB-1 mucin 1 having α2,8-disialyl residue obtained in Example 1 was diluted between 10 and 6000 times with a diluent wherein 1% BSA had been added to PBS-0.1% Tween-20 (PBS-T). Then, 50µL of the diluted YMB-1 mucin 1 solution was added to the wells, and incubated at 25 °C for 2 hrs. After washing with PBS-T three times, 50µL of KL-6 antibody diluted by a factor of 1000 was added and incubated at 25°C for 1.5hr. The wells were washed with PBS-T four times, and 100µL of SuperSignal ELISA Pico Chemiluminescent Substrate (PIERCE, Rockford, IL) was added and chemiluminescent quantification was carried out by using a Plate CHAMELEON V (HIDEX Oy, Turku, Finland).

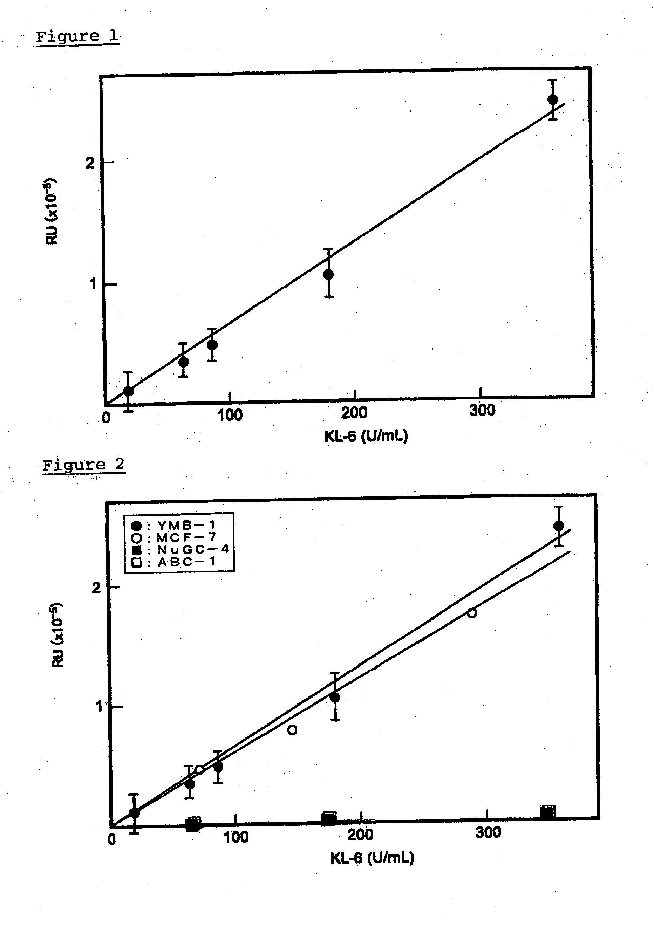

The obtained standard curve is shown in Figure 1. The diluted YMB-1 mucin 1 solutions were further measured by a KL-6 measurement kit (Eitest KL-6: Eizai Co., Ltd), to calculate measured values (units) of the YMB-1 mucin 1 solution. The amount of mucin 1 having α2,8-disialyl residue in a sample was calculated from the measured values (units) of the YMB-1 mucin 1 solution as a standard.

### <<Reference Example 3: Measurement of mucin 1 in extract from cancer cell lines >>

In this Reference Example, expressions of mucin 1 having α2,8-disialyl residue in cancer cell lines other than breast cancer were examined.

The procedure described in Reference Example 1 was repeated except that breast cancer cell line MCF-7, gastric cancer cell line NUGC-4, and lung cancer cell line ABC-1 were used instead of the breast cancer cell line YMB-1, to obtain MCF-7 mucin 1 solution, NuGC-4 mucin 1 solution, and ABC-1 mucin 1 solution. Mucin 1 having α2,8-disialyl residue in the above mucin 1 solutions were measured by the sandwich assay described in Reference Example 2.

The results are shown in Figure 2. In the mucin 1 solutions of the breast cancer cell line YMB-1 and the breast cancer cell line MCF-7, mucin 1 having α2,8-disialyl residue was detected. However, mucin 1 having α2,8-disialyl residue was not detected in the mucin 1 solutions of the gastric cancer cell line NUGC-4, and the lung cancer cell line ABC-1.

### <Reference Example 4: Measurement of sera of breast cancer patients>>

The amount of mucin 1 having α2,8-disialyl residue was measured in sera of 10 breast cancer patients, which showed high levels of CA15-3, sera of 10 healthy subjects and sera of 30 interstitial pneumonia patients.

The procedure described in Reference Example 2 was repeated except that sera of 10 breast cancer patients, sera of 10 healthy subjects and sera of 30 interstitial pneumonia patients were used instead of the YMB-1 mucin 1 solution. The measured value (units) in each sample was calculated from the standard curve obtained in Reference Example 2.

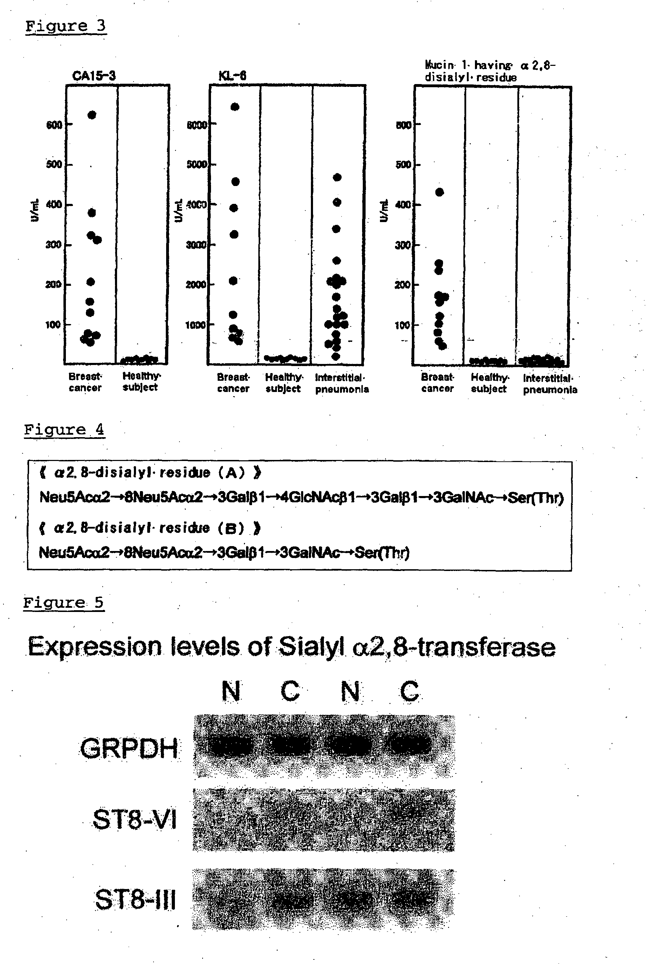

The results are shown in Figure 3. In the sera of healthy subjects and sera of interstitial pneumonia patients, mucin 1 having α2,8-disialyl residue cannot be detected. On the other hand, mucin 1 having α2,8-disialyl residue can be detected in all ten sera of breast cancer patients.

### <<Comparative Example 1: Measurement of KL-6 and CA15-3>>

In this Example, the amount of KL-6, which is the conventional marker for interstitial pneumonia, was measured, in sera of 10 breast cancer patients, sera of 10 healthy subjects and sera of 30 interstitial pneumonia patients. Further, the amount of CA15-3, which is the tumor marker for breast cancer, was measured in sera of 10 breast cancer patients, and sera of 10 healthy subjects.

The measurement of KL-6 was carried out using the "Eitest KL-6" (Eizai Co., Ltd) in accordance with the protocol attached to thereto.

The measurement of CA15-3 was carried out using the "E test TOSOH II (CA15-3)" (TOSOH Co., Ltd) in accordance with the protocol attached to thereto.

The result is shown in Figure 3. In the 10 breast cancer patients, the amount of CA15-3 was about 50 to 650units/mL, and the amount of KL-6 was about 500 to 6500 units/mL. In addition, KL-6 is detected in sera of all the 30 interstitial pneumonia patients.

The measured values of mucin 1 having α2,8-disialyl residue, CA15-3, and KL-6 in 10 breast cancer patients are shown in Table 1.

**Table 1**

| **Sample· No.** | **Mucin·1·having· α2,8-disialyl· residue· (Units/mL)** | **KL-6 (Unit/mL)** | **CA 15-3 (Unit/mL)** |
|---|---|---|---|
| **1** | **122.0** | **1278** | **137.8** |
| **2** | **178.0** | **3960** | **329.2** |
| **3** | **57.2** | **2200** | **207.2** |
| **4** | **171.0** | **828** | **66.5** |
| **5** | **78.1** | **3580** | **380.3** |
| **6** | **46.8** | **784** | **76.9** |
| **7** | **164.0** | **3240** | **316.2** |
| **8** | **266.0** | **704** | **58.2** |
| **9** | **249.0** | **648** | **77.5** |
| **10** | **431.0** | **6960** | **637.2** |

### <<Reference Example 5>>

In this Reference Example, the α2,8-disialyl residue of mucin 1 of breast cancer cells was analyzed. Sugar chains of mucin 1 expressed by breast cancer cells were released by β-elimination, and then were reduced and labeled by NaB³H₄. The resulting sugar chains were fractionated by high-voltage paper electrophoresis at pH 5.4. Each resulting fraction was analyzed by Bio-Gel P-4 column chromatography, various lectin column chromatographies, and/or exo-glycosidase (including sialidase) digestion, so as to determine a carbohydrate structure of the sugar chain structure in each fraction.

As a result, about 9 mole% of Neu5Acα2→8Neu5Acα2→3Galβ1→4GlcNAcβ1→3Galβ1→3GalNAc→Ser(Thr), and about 1 mole% of Neu5Acα2→8Neu5Acα2→3Galβ1→3GalNAc→Ser(Thr) as the α2,8-disialyl residue are found to be contained in mucin 1 (Figure 4).

### <<Reference Example 6>>

In this Reference Example, α2,8-sialyltransferase capable of adding α2, 8-disialyl residue to mucin 1 of breast cancer patients was identified. Further, the expression levels of these α2,8-sialyltransferase in breast cancer tissues were confirmed.

First of all, mRNA expression levels of α2,8-sialyltransferase I (ST8-I), α2,8-sialyltransferase II (ST8-II), α2,8-sialyltransferase III (ST8-III), α2,8-sialyltransferase IV (ST8-IV), α2,8-sialyltransferase V (ST8-V), and α2,8-sialyltransferase VI (ST8-VI) in 4 cases of human breast cancer tissues and lacteal gland tissues of healthy humans were examined by a real-time polymerase chain reaction (hereinafter, referred to as a real time PCR). Total RNA was prepared from the tissues of human breast cancer and healthy human lacteal gland using ISOGEN (NIPPON GENE CO., LTD), and then the total RNA was extracted with chloroform/isopropyl alcohol. Extracted total RNA was precipitated with ethanol, and then dissolved in diethyl carbonate-treated distilled water. The total RNA was subjected to a reverse transcription reaction with oligo(dT)primer using Superscript III (Invitrogen Corporation), to obtain CDNA. The real-time PCR was performed by a Dice (registered trademark) real-time system (TP800, Takara Bio Inc.) using Power SYBR (registered trademark) Green PCR master mix (Life Technologies), and primers that were gene-specific to each α2,8-sialyltransferase.

The primers of each α2,8-sialyltransferase used are as follows:
ST8-I:5'-TTCAACTTACTCTCTCTTCCCACA-3' (SEQ ID NO: 5), and 5'-TCTTCTTCAGAATCCCACCATT-3'(SEQ ID NO: 6)(GenBank accession no.6489);
ST8-II:5'-CTCAGAGATCGAAGAAGAAATCG-3'(SEQ ID NO: 7), and 5'-GCTGTTCACAGCTGATCTGAT-3' (SEQ ID NO: 8)(GenBank accession no.8128);
ST8-III:5'-CAGGTACCCACAAAACAGTGC-3' (SEQ ID NO: 9), and 5'-GAGCTTACTGGGTGCCTTGT-3' (SEQ ID NO: 10) (GenBank accession no.51046);
ST8-IV:5'-AATGTGGAAAGGAGATTGACAGT-3' (SEQ ID NO: 11), and 5'-TCTGATTTAGTTCCCACATCTGC-3'(SEQ ID NO: 12)(GenBank accession no.7903);
ST8-V:5'-GCTGAGGCACGAAATATTGG-3'(SEQ ID NO: 13), and 5'-TGTCGAACAGCTCTGACTGC-3'(SEQ ID NO: 14)(GenBank accession no.29906);
ST8-VI: 5'-GGCAAGCAGAAGAATATGCAA-3' (SEQ ID NO: 3), and 5'-AAACAACAAAGTTTTGAACAGCAT-3' (SEQ ID NO: 4) (GenBank accession no.338596).

Normalization of mRNA expression was performed using GAPDH: 5'-ATCCACATCGCTCAGACAC-3' (SEQ ID NO: 15), and 5'-GCCCAATACGACCAAATCC-3' (SEQ ID NO: 16) (GenBank accession no. NM_002046) as internal standard primers.

The real-time PCR program repeated 40 cycles of 95°C, 10 seconds and 60°C, 40 seconds. A single sharp peak was obtained by the respective primer set, and the specific PCR product was amplified, and no primer dimer was found. The tests were repeated three times for each of the samples.

The mRNA expression levels of ST8-III and ST8-VI in human breast cancer tissue were found to be higher than those in healthy human lacteal gland tissues.

Further, in order to confirm the expression of mRNA of ST8-III and ST8-VI in the above 4 cases, CDNA was synthesized, and then PCR performed using forward primer and reverse primer of ST8-III or ST8-VI. The PCR program repeated 50 cycles of 95°C, 30 seconds; 52. 5°C, 30 seconds; and 72°C, 30 seconds. Resulting PCR products were analyzed by an electrophoresis to confirm the expression levels of mRNA of ST8-III and ST8-VI. As a control, RT-PCR as to GAPDH was performed. The results are shown in Figure 5.

The expression level of ST8-VI mRNA in human breast cancer tissue was increased about 10 times, compared to normal human lacteal gland tissue. This result indicates that ST8-VI relates to the increase of the α2,8-disialyl residue in mucin 1 of the breast cancer.

### INDUSTRIAL APPLICABILITY

By applying the method for analyzing mucin 1 having α2,8-disialyl residue, it is possible to easily distinguish breast cancer from interstitial pneumonia.

Although the present invention has been described with reference to specific embodiments, various changes and modifications obvious to those skilled in the art are possible without departing from the scope of the appended claims.

## Claims

1. A method for distinguishing breast cancer from interstitial pneumonia **characterized by** analyzing mucin 1 having Siaα2-8Siaα2-3Galβ-R, wherein the method comprises the step of bringing a first probe specifically binding to a mucin 1 having Siaα2-8Siaα2-3Galp-R into contact with a sample to be tested.

2. The method for distinguishing breast cancer from interstitial pneumonia according to claim 1, wherein the method comprises steps of:
bringing the first probe specifically binding to the mucin 1 having Siaα2-8Siaα2-3Galβ-R into contact with the sample to be tested; and
detecting a bound complex of the first probe and the mucin having Siaα2-8Siaα2-3Galβ-R.

3. The method for distinguishing breast cancer from interstitial pneumonia according to claim 1 or 2, wherein the method Is a sandwich assay which comprises the steps of:
bringing the first probe specifically binding to the mucin 1 having Siaα2-8Siaα2-3Galβ-R into contact with the sample to be tested;
bringing a second probe specifically binding to the mucin 1 having Siaα2-8Siaα2-3Galβ-R into contact with the sample to be tested; and
detecting the bound complex of the first probe and the mucin 1 having Siaα2-8Siaα2-3Galβ-R.

4. The method for distinguishing breast cancer from interstitial pneumonia according to any one of claims 1 to 3, wherein the first probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or a mixture thereof.

5. The method for distinguishing breast cancer from interstitial pneumonia according to claim 3, wherein
the first probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or a mixture thereof; and the second probe is an antibody specifically binding to Siaα2-8Siaα2-3Galβ-R, or an antibody fragment having the antigen-binding site thereof; an antibody specifically binding to the mucin 1 having Siaα2-8Siaα2-3Galβ-R, or an antibody fragment having the antigen-binding site thereof; or an antibody specifically binding to the mucin 1, or an antibody fragment having the antigen-binding site thereof; or a mixture of two or more thereof.

## Patentansprüche

1. Verfahren zur Unterscheidung von Brustkrebs von interstitieller Pneumonie, **gekennzeichnet durch** die Analyse von Mucin-1 mit Siaα2-8Siaα2-3Galβ-R, wobei das Verfahren den Schritt des Inkontaktbringens einer ersten Sonde, welche spezifisch an ein Mucin-1 mit Siaα2-8Siaα2-3Galβ-R bindet, mit einer Probe, welche getestet werden soll, umfasst.

2. Verfahren zur Unterscheidung von Brustkrebs von interstitieller Pneumonie nach Anspruch 1, worin das Verfahren die Schritte umfasst:
Inkontaktbringen der ersten Sonde, welche spezifisch an das Mucin-1 mit Siaa2-8Siaa2-3Galβ-R bindet, mit der Probe, welche getestet werden soll;
und
Detektieren eines gebundenen Komplexes der ersten Sonde und des Mucin-1 mit Siaa2-8Siaα2-3Galβ-R.

3. Verfahren zur Unterscheidung von Brustkrebs von interstitieller Pneumonie nach einem der Ansprüche 1 oder 2, worin das Verfahren ein Sandwich-Test ist, welcher die Schritte umfasst:
Inkontaktbringen der ersten Sonde, welche spezifisch an das Mucin-1 mit Siaa2-8Siaa2-3Galβ-R bindet, mit der Probe, welche getestet werden soll;
Inkontaktbringen einer zweiten Sonde, welche spezifisch an das Mucin-1 mit Siaa2-8Siaα2-3Galβ-R bindet, mit der Probe, welche getestet werden soll;
und
Detektieren des gebundenen Komplexes der ersten Sonde und des Mucin-1 mit Siaa2-8Siaα2-3Galβ-R.

4. Verfahren zur Unterscheidung von Brustkrebs von interstitieller Pneumonie nach einem der Ansprüche 1 bis 3, worin die erste Sonde ein Antikörper, welcher spezifisch an Siaα2-8Siaα2-3Galβ-R bindet, oder ein Antikörperfragment mit der Antigen-Bindungsstelle davon, ist; ein Antikörper, welcher spezifisch an das Mucin-1 mit Siaa2-8Siaα2-3Galβ-R bindet, oder ein Antikörperfragment mit der Antigen-Bindungsstelle davon, ist; oder eine Mischung davon ist.

5. Verfahren zur Unterscheidung von Brustkrebs von interstitieller Pneumonie nach Anspruch 3, worin die erste Sonde ein Antikörper, welcher spezifisch an Siaa2-8Siaa2-3Galβ-R bindet, oder ein Antikörperfragment mit der Antigen-Bindungsstelle davon, ist; ein Antikörper, welcher spezifisch an das Mucin-1 mit Siaα2-8Siaα2-3Galβ-R bindet oder ein Antikörperfragment mit der Antigen-Bindungsstelle davon, ist; oder eine Mischung davon ist; und die zweite Sonde ein Antikörper, welcher spezifisch an Siaa2-8Siaa2-3Galβ-R bindet, oder ein Antikörperfragment mit der Antigen-Bindungsstelle davon, ist; ein Antikörper, welcher spezifisch an das Mucin-1 mit Siaα2-8Siaα2-3Galβ-R bindet, oder ein Antikörperfragment mit der Antigen-Bindungsstelle davon, ist; oder ein Antikörper, welcher spezifisch an das Mucin-1 bindet, oder ein oder ein Antikörperfragment mit der Antigen-Bindungsstelle davon, ist; oder eine Mischung von zwei oder mehreren davon ist.

## Revendications

1. Procédé pour distinguer le cancer du sein de la pneumonie interstitielle **caractérisé par** l'analyse de la mucine-1 ayant de Siaα-8Siaα2-3Galβ-R, dans lequel le procédé comprend l'étape de la mise en contact d'une première sonde qui se combine spécifiquement avec une mucine-1 ayant de Siaα2-8Siaα2-3Galβ-R avec un échantillon à tester.

2. Procédé pour distinguer le cancer du sein de la pneumonie interstitielle selon la revendication 1, dans lequel le procédé comprend les étapes :
de la mise en contact de la première sonde qui se combine spécifiquement avec la mucine-1 ayant de Siaα2-8Siaα2-3Galβ-R avec l'échantillon à tester ;
et
de la détection d'un complexe combiné de la première sonde et de la mucine-1 ayant de Siaα2-8Siaα2-3Galβ3-R.

3. Procédé pour distinguer le cancer du sein de la pneumonie interstitielle selon l'une des revendications 1 ou 2, dans lequel le procédé est un test en sandwich qui comprend les étapes :
de la mise en contact de la première sonde qui se combine spécifiquement avec la mucine-1 ayant de Siaα2-8Siaα2-3Galβ-R avec l'échantillon à tester ;
de la mise en contact d'une deuxième sonde qui se combine spécifiquement avec la mucine-1 ayant de Siaα2-8Siaα2-3Galβ-R avec l'échantillon à tester ;
et
de la détection du complexe combiné de la première sonde et de la mucine-1 ayant de Siaα2-8Siaα2-3Galβ-R.

4. Procédé pour distinguer le cancer du sein de la pneumonie interstitielle selon l'une des revendications 1 à 3, dans lequel la première sonde est un anticorps qui se combine spécifiquement avec du Siaα2-8Siaα2-3Ga1β-R ou un fragment de l'anticorps avec son site de liaison de l'antigène ; un anticorps qui se combine spécifiquement avec la mucine-1 ayant de Siaα2-8Siaα2-3Galβ-R ou un fragment de l'anticorps avec son site de liaison de l'antigène ; ou un mélange de ceux-ci.

5. Procédé pour distinguer le cancer du sein de la pneumonie interstitielle selon la revendication 3, dans lequel la première sonde est un anticorps qui se combine spécifiquement avec du Siaα2-8Siaα2-3Galβ-R ou un fragment de l'anticorps avec son site de liaison de l'antigène ; un anticorps qui se combine spécifiquement avec la mucine-1 ayant de Siaα2-8Siaα2-3Galβ-R ou un fragment de l'anticorps avec son site de liaison de l'antigène ; ou un mélange de ceux-ci ; et
la deuxième sonde est un anticorps qui se combine spécifiquement avec du Siaa2-8Siaα2-3Galβ-R ou un fragment de l'anticorps avec son site de liaison de l'antigène ; un anticorps qui se combine spécifiquement avec la mucine-1 ayant de Siaa2-8Siaa2-3Galβ-R ou un fragment de l'anticorps avec son site de liaison de l'antigène ; ou un anticorps qui se combine spécifiquement avec la mucine-1 ou un fragment de l'anticorps avec son site de liaison de l'antigène ; ou un mélange de deux ou plusieurs de ceux-ci.
